# EUROPÄISCHE PATENTSCHRIFT

(11) **EP 0 632 130 B1**
(45) Veröffentlichungstag und Bekanntmachung des Hinweises auf die Patenterteilung: **23.06.1999**
(21) Anmeldenummer: 94107742.2
(22) Anmeldetag: 19.05.1994
(51) Int. Cl.: C12P 13/00

(54) **Enzymatisches Verfahren zur Herstellung aliphatischer S-Cyanhydrine**
Enzymatic process for the production of aliphatic S-cyanhydrins
Procédé enzymatique de la production de S-cyanhydrines aliphatiques

(30) Priorität: 01.06.1993 AT 105593
(43) Veröffentlichungstag der Anmeldung: 04.01.1995
(73) Patentinhaber: DSM Fine Chemicals Austria GmbH, 4021 Linz (AT)
(72) Erfinder: Griengl, Herfried, Dr., A-8047 Graz (AT); Shi, Nongyuan, Dr., A-8010 Graz (AT); Pichler, Ulrike, Dipl.-Ing., A-8010 Graz (AT); Klempier, Norbert, Dr., A-8044 Graz (AT); Pöchlauer, Peter, Mag. Dr., A-4020 Linz (AT)
(74) Vertreter: Klostermann, Ingrid

(56) Entgegenhaltungen:
- EP-A- 0 326 063
- EP-A- 0 350 908
- EP-A- 0 539 767
- DE-A- 4 028 689

## Beschreibung

Cyanhydrine sind etwa zur Synthese von alpha-Hydroxysäuren, alpha-Hydroxyketonen, beta-Aminoalkoholen, die zur Gewinnung biologisch wirksamer Stoffe, z.B. pharmazeutischer Wirkstoffe, Vitamine oder auch pyrethroider Verbindungen Verwendung finden, von Bedeutung.

Die Herstellung eines Cyanhydrins kann durch Anlagerung einer Cyanidgruppe an den Carbonylkohlenstoff eines Aldehyds oder eines unsymmetrischen Ketons erfolgen, wobei Enantiomerengemische optisch aktiver Cyanhydrine entstehen.

Da in einem biologisch wirksamen Enantiomerengemisch normalerweise nur eines der beiden Enantiomere biologisch wirksam ist, hat es nicht an Versuchen gefehlt, ein Verfahren zur Herstellung des S-Enantiomeren eines optisch aktiven Cyanhydrins in möglichst hoher optischer Reinheit zu finden.

So ist etwa in Makromol. Chem. 186, (1985), 1755-62 ein Verfahren zur Gewinnung von S-Cyanhydrinen durch Umsetzung von Aldehyden mit Blausäure in Gegenwart von Benzyloxycarbonyl-(S)-phenylalanin-(S)-histidinmethylester als Katalysator beschrieben. Die optische Reinheit der erhaltenen S-Cyanhydrine ist aber höchst unbefriedigend.

In EP-A-0 326 063 ist ein enzymatisches Verfahren zur Herstellung von optisch aktiven (R)- oder (S)-Cyanhydrinen durch Umsetzung von aliphatischen, aromatischen oder heteroaromatischen Aldehyden oder Ketonen mit Blausäure in Gegenwart von (R)-Oxynitrilase (4.1, 2.10) aus Prunus amygdalis oder Oxynitrilase (4.1,2.11) aus Sorghum bicolor beschrieben. Beispiele für die stereospezifische Herstellung von aliphatischen (S)-Cyanhydrinen sind nicht angegeben. Dies ist nicht verwunderlich, denn in Angew. Chemie 102 (1990), Nr. 4, pp 423-425 ist von Erfindern, die in EP-A-0 326063 genannt sind, angegeben, daß von der (S)-Oxynitrilase aus Sorghum bicolor keine aliphatischen S-Cyanhydrine mit Blausäure hergestellt werden können. Dieser Befund wird auch durch F. Effenberger et al. in Tetrahedron Letters Vol. 31, No. 9 (1990), pp. 1249-1252 bestätigt.

Aus EP - A 0539 767 ist ein enzymatisches Verfahren zur Herstellung aliphatischer (S)-Cyanhydrine durch Umsetzung eines aliphatischen Aldehyds oder eines unsymmetrischen Ketons mit einem Cyanhydrin als Cyanidgruppendonor in einem Verdünnungsmittel in Gegenwart von (S)-Oxynitrilase aus Hevea brasiliensis. Die gemäß dieser Anmeldung bei Raumtemperatur unter Verwendung eines Cyanhydrin als Cyanidgruppendonor hergestellten (S)-Cyanhydrine können in relativ guter Ausbeute und akzeptabler optischer Reinheit erhalten werden.

Aufgabe der Erfindung war es ein hinsichtlich Ausbeute und optischer Reinheit der Produkte verbessertes enantioselektives enzymatisches Verfahren zur Herstellung aliphatischer (S)-Cyanhydrine bereitzustellen, bei dem ein aliphatischer Aldehyd oder ein unsymmetrisches aliphatisches Keton in Gegenwart von Hevea brasiliensis umgesetzt wird.

Es wurde nun unerwarteterweise gefunden, daß es doch möglich ist, aliphatische Aldehyde oder unsymmetrische aliphatische Ketone mit Blausäure und Oxynitrilase stereospezifisch zu S-Cyanhydrinen umzusetzen, wenn man als Enzym die (S)-Oxynitrilase aus Hevea brasiliensis einsetzt und die Reaktion bei Temperaturen bis zu höchstens 10 °C ausführt: Die dabei erzielten Ausbeuten und die optische Reinheit sind gegenüber dem Stand der Technik erheblich verbessert.

Gegenstand der Erfindung ist daher ein enantioselektives enzymatisches Verfahren zur Herstellung aliphatischer (S)-Cyanhydrine in Gegenwart der (S)-Oxynitrilase aus Hevea brasiliensis das dadurch gekennzeichnet ist, daß ein aliphatischer Aldehyd oder ein unsymmetrisches aliphatisches Keton mit Blausäure in einem Verdünnungsmittel bei Temperaturen bis höchstens 10 °C umgesetzt wird.

Als Ausgangsmaterialien im erfindungsgemäßen Verfahren werden ein aliphatischer Aldehyd oder ein unsymmetrisches aliphatisches Keton, Blausäure, Oxynitrilase aus Hevea brasiliensis und ein Verdünnungsmittel eingesetzt.

Unter Aldehyden sind dabei gesättigte oder ungesättigte aliphatische, geradkettige, verzweigte oder cyclische Aldehyde zu verstehen. Insbesonders eignet sich das Verfahren zur Umsetzung geradkettiger Aldehyde mit insbesonders 2 bis 18 C-Atomen, bevorzugt von 2 bis 12, die gesättigt oder ein- oder mehrfach ungesättigt sind. Der Aldehyd kann dabei sowohl C-C-Doppelbindungen als auch C-C-Dreifachbindungen aufweisen. Der Aldehyd kann unsubstituiert oder durch unter den Reaktionsbedingungen inerte Gruppen, beispielsweise durch gegebenenfalls substituierte Aryl- oder Heteroarylgruppen wie Phenyl- oder Indolylgruppen, durch Halogen-, Ether-, Alkohol-, Acyl-, Carbonsäure-, Carbonsäureester-, Nitro- oder Azidogruppen substituiert sein. Bevorzugt ist der Aldehyd unsubstituiert.
Unter Ketonen sind gesättigte oder ungesättigte, geradkettige, verzweigte oder cyclische Ketone zu verstehen. Bevorzugt sind dabei Ketone, die an einer Seite möglichst wenig sterisch gehindert sind, insbesondere Methylketone. Die Ketone können gesättigt oder ein- oder mehrfach ungesättigt sein. Sie können unsubstituiert, oder durch unter Reaktionsbedingungen inerte Gruppen, beispielsweise durch gegebenenfalls substituierte Aryl- oder Heteroarylgruppen wie Phenyl- oder Indolylgruppen, durch Halogen-, Ether-, Alkohol-, Acyl-, Carbonsäure-, Carbonsäure-ester-, Nitro- oder Azidogruppen substituiert sein.

Als Cyanidgruppendonor wird Blausäure eingesetzt. Die Blausäure kann als Gas zugeleitet oder in Form einer ihrer Salze wie NaCN oder KCN eingesetzt werden, wobei sie durch die gegebenen Reaktionsbedingungen im Zuge der Reaktion in situ daraus freigesetzt wird.

Als Oxynitrilase wird die S-Oxynitrilase aus Hevea brasiliensis verwendet. Sie kann gereinigt oder ungereinigt, als solche oder immobilisiert eingesetzt werden. Ihre Bereitstellung und Reinigung kann beispielsweise durch Fällung mit Ammoniumsulfat und anschließender Dialyse etwa gemäß D. Selmar et al., Physiologia Plantarium 75 (1989), 97-101 erfolgen.

Die Umsetzung erfolgt in einem Verdünnungsmittel. Als besonders vorteilhaft hat sich herausgestellt, daß die Umsetzung in einem wäßrigen Verdünnungsmittel ohne Zusatz organischer Lösungsmittel, die die Aktivität des Enzyms rasch hemmen können, erfolgen kann, wobei es unerwarteterweise zu keiner Racemisierung des Produktes kommt. Die erfindungsgemäß Umsetzung kann aber auch in einem organischen Verdünnungsmittel oder in Gegenwart eines organischen Lösungsmittels für den Aldehyd oder das Keton gegebenenfalls in einem Zweiphasensystem, beispielsweise in einem Membranreaktor, erfolgen. Als organische Verdünnungsmittel oder Lösungsmittel können aliphatische oder aromatische Kohlenwasserstoffe, die gegebenenfalls halogeniert sind, Alkohole, Ether, Ester verwendet werden. Als organisches Lösungsmittel für den Aldehyd oder das Keton im Zweiphasensystem können mit Wasser nicht mischbare organische Lösungsmittel wie z.B. aliphatische oder aromatische Kohlenwasserstoffe, die gegebenenfalls halogeniert sind, Ether, Ester eingesetzt werden. Bevorzugt erfolgt die Umsetzung nicht in Anwesenheit eines organischen Lösungsmittel sondern in einem wäßrigen Verdünnungsmittel. Als wäßriges Verdünnungsmittel wird Wasser, eine wäßrige Salz- oder eine wäßrige Pufferlösung eingesetzt. Bevorzugt wird eine wäßrige Pufferlösung, ganz bevorzugt eine solche, die Natriumcitrat enthält, verwendet. Der pH-Wert soll dabei unter 7, bevorzugt etwa 3 bis 5 betragen.

Pro Mol Aldehyd oder Keton werden etwa 1.500 bis 15.000 g Verdünnungsmittel und 10⁴ bis 10⁶ IU Aktivität Oxynitrilase, bevorzugt etwa 10⁵ bis 5.10⁵ IU, zugesetzt. Ein IU (International Unit) drückt dabei die Bildung von einem Mikromol Produkt pro Minute und pro Gramm Enzymrohisolierung aus. Die benötigte Menge der Oxynitrilase ermittelt man am besten in einem Aktivitätstest, etwa gemäß Selmar et al., Analytical Biochemistry 166 (1987), 208-211.

Pro Mol eingesetzte Aldehyd- oder Ketogruppe werden mindestens ein Mol, bevorzugt 1 bis 3 Mol Blausäure zugegeben. Bei Zugabe als Salz wird die Blausäure infolge des pH-Wertes, der unter 7 beträgt, aus dem Salz freigesetzt.

Die Reaktionsmischung wird bei Temperaturen von etwa -5 bis 10 °C geschüttelt oder gerührt. Es hat sich unerwarteterweise herausgestellt, daß die Oxynitrilase am besten in einem Temperaturbereich von -5 bis 5 °C eingesetzt wird. Trotz der tiefen Temperatur zeigt das Enzym dabei so gut wie keine Aktivitätsverluste.
Dabei wird die Cyanidgruppe der Blausäure auf das Carbonylkohlenstoffatom des eingesetzten Aldehyds oder Ketons übertragen und es entsteht überwiegend das S-Enantiomere des, dem eingesetzten Aldehyd oder Keton entsprechenden, optisch aktiven Cyanhydrins. Der Fortschritt der Reaktion wird dabei beispielsweise gaschromatographisch verfolgt.
Nach erfolgter Umsetzung kann das gebildete Cyanhydrin aus der Reaktionsmischung mit Hilfe eines organischen Lösungsmittels, das mit Wasser nicht mischbar ist, etwa aliphatische oder aromatische gegebenenfalls halogenierte Kohlenwasserstoffe, z.B.
Pentan, Hexan, Toluol, Xylole, Methylenchlorid, Ether wie etwa Diethylether, Diisopropylether, Methyl-tert.Butylether, oder Ester, beispielsweise Essigsäureethylester oder Mischungen solcher Lösungsmittel extrahiert werden. Sollte die Reinheit des extrahierten Produktes nicht ausreichend sein, kann eine Reinigungsoperation angeschlossen werden. Die Reinigung kann durch eine bekannte Methode erfolgen und gelingt am besten chromatographisch.

In einer bevorzugten Ausführungsform werden etwa 100 mg Aldehyd oder Keton in 15 bis 30 g einer wäßrigen Pufferlösung mit einem pH-Wert von etwa 4, die Natriumcitrat enthält, mit 2 Mol Blausäure pro Mol eingesetzter Aldehyd- oder Ketogruppe und 10⁵ bis 5.10⁵ IU Aktivität Oxynitrilase aus Hevea brasiliensis bei Temperaturen von 0 bis 5 °C im geschlossenen System geschüttelt oder gerührt. Der Fortschritt der Reaktion wird gaschromatographisch verfolgt. Nach beendeter Reaktion wird die Reaktionsmischung mit Methylenchlorid extrahiert, die organische Phase getrocknet und abgedampft. Eine weitere Reinigung des Rückstandes kann säulenchromatogrphisch erfolgen.

Auf die beschriebene Art und Weise werden auf einfache Weise erstmals optisch aktive S-angereicherte Cyanhydrine aus aliphatischen Aldehyden oder unsymmetrischen aliphatischen Ketonen durch Umsetzen mit Blausäure in guten Ausbeuten und guter Reinheit erhalten. Das Verfahren stellt daher eine Bereicherung der Technik dar.

### Beispiel 1

500 IU Enzymrohpräparierung aus Hevea brasiliensis wurden in 20 ml 0,5 n Natriumcitratpufffer, pH = 3,75 suspendiert, mit 200 mg Hexanal (2 mmol) versetzt und auf 0 °C gekühlt. Anschließend wurden unter Rühren 260 mg KCN (4 mmol) in 20 ml 0,5 n Natriumcitratpuffer, pH = 3,75 gelöst und innerhalb von 30 Minuten eingetropft. Nach beendeter Umsetzung, die gaschromatographisch festgestellt wurde, wurde die wäßrige Phase zwei Mal mit je 25 ml Methyl-tert.Butylether extrahiert, die vereinigten organischen Phasen über Na₂SO₄ getrocknet und abgedampft. Der Rückstand wurde über Kieselgel mit Petrolether : Essigsäureethylester = 4 : 1 chromatographiert. Dabei wurden 0,22 g, das sind 86 % der Theorie, S-Hexanalcyanhydrin, mit einem [α] D²⁰ = -13,4°, das entspricht einer optischen Reinheit ee von 92 %, erhalten.
Die Bestimmung der optischen Reinheit erfolgte gemäß V. Schuring et al., Ang. Chemie 102 (1990), 969-986 durch gaschromatographische Analyse des mit (S)-Menthylchlorformiat derivatisierten Cyanhydrins auf einer Polysiloxantrennsäule (30 m x 0,25 mm, 86 % Polydimethylsiloxan, 7 % Cyanopropylsiloxan, 7 % Phenylsiloxan).
Die Derivatisierung wurde wie folgt vorgenommen: 5-10 mg des Hexanalcyanhydrins wurden mit dreimolarem Überschuß (S)-Menthylchlorformiat und Pyridin in trockenem Methylenchlorid in einem Derivatisierungsgefäß vereinigt und nach 10 Minuten gaschromatographisch analysiert.

### Beispiel 2

Entsprechend Beispiel 1 wurden 68 mg (1 mmol) Propenal mit 130 mg (2 mmol) KCN und 200 IU Enzymrohpräparierung aus Hevea brasiliensis in insgesamt 15 ml 0,5 n Natriumcitratpuffer pH 3,75 umgesetzt. Die Isolierung des Propenalcyanhydrins erfolgte nach 2 Stunden wie in Beispiel 1 beschrieben. Dabei wurden 0,029 g, das sind 38 % der Theorie, (S)-Propenalcyanhydrin, mit einem [α] D²⁰ = + 4,9, das entspricht einer optischen Reinheit (ee) von 91 %, erhalten.

### Beispiel 3

Entsprechend Beispiel 1 wurden 196 mg (2 mmol) 2-(E)-Hexenal mit 260 mg (4 mmol) KCN und 700 IU Enzymrohpräparierung aus Hevea brasiliensis in insgesamt 25 ml 0,5 n Natriumcitratpuffer ph 3,75 umgesetzt. Die Isolierung des 2-(E)-Hexenalcyanhydrins erfolgte nach 2 Stunden wie in Beispiel 1 beschrieben. Dabei wurden 0,043 g, das sind 17 % der Theorie, (S)-2-(E)-Hexenalcyanhydrin, mit einem [α] D²⁰ = + 20,3, das entspricht einer optischen Reinheit (ee) von 96 %, erhalten.

### Beispiel 4

Entsprechend Beispiel 1 wurden 144 mg (1,5 mmol) 2-Hexinal mit 195 mg (3 mmol) KCN und 600 IU Enzymrohpräparierung aus Hevea brasiliensis in insgesamt 25 ml 0,5 n Natriumcitratpuffer pH 3,75 umgesetzt. Die Isolierung des 2-Hexinalcyanhydrins erfolgte nach 3 Stunden wie in Beispiel 1 beschrieben. Dabei wurden 0,081 g, das sind 43 % der Theorie, (S)-2-Hexinalcyanhydrin, mit einem [α] D²⁰ = - 20,4, das entspricht einer optischen Reinheit (ee) von 80 %, erhalten.

### Beispiel 5

Entsprechend Beispiel 1 wurden 98 mg (1,4 mmol) 2-(E)-Butenal mit 182 mg (2,8 mmol) KCN und 200 IU Enzymrohpräparierung aus Hevea brasiliensis in insgesamt 20 ml 0,5 n Natriumcitratpuffer pH 3,75 umgesetzt. Die Isolierung des 2-(E)-Butenalcyanhydrins erfolgte nach 3 Stunden wie in Beispiel 1 beschrieben. Dabei wurden 0,038 g, das sind 28 % der Theorie, (S)-2-(E)-Butenalcyanhydrin, mit einem [α] D²⁰ = + 29,4, das entspricht einer optischen Reinheit (ee) von 87 %, erhalten.

### Beispiel 6

2-Pentanon (3,0 mmol, 258 mg) und Enzymrohpräparierung aus Hevea brasiliensis (168 mg, 200 IU) wurden in 0,1 M Natriumcitrat (ph 3,75, 5 ml) suspendiert. Unter Eiskühlung wurde eine Lösung von Kaliumcyanid (6,0 mmol, 391 mg) in 0,1 M Citronensäure (50 ml) 20 Min. lang zugetropft und danach mit 0,1 M Citronensäure auf pH 4 eingestellt. Die Reaktionslösung wurde unter Eiskühlung 3,5 h gerührt, mit Diethylether extrahiert und über Na₂SO₄ getrocknet. Nach Abdestillieren des Lösungsmittels wurde der Rückstand chromatographisch (Kieselgel, Essigsäureester/Benzin 1/6) gereinigt. 100 mg (30 %) (S)-2-Hydroxy-2-methylpentannitril wurden erhalten. ee: 75 %. [α] D²⁰ = - 2,5° (c=2, CHCl₃).

### Beispiel 7

Analog wurden aus 3-Methyl-2-butanon (4,1 mmol, 352 mg), Enzymrohpräparierung aus Hevea brasiliensis (200 IU, 168 mg), KCN (8,4 mmol, 546 mg), Na-Citrat (0,1 M, ph 3,75, 5 ml) und Citronensäure (0,1 M, 55 ml) 144 mg, das sind 30 % der Theorie, (S)-2-Hydroxy-2,3-dimethyl-butannitril erhalten.
ee: 82 %. [α] D²⁰ = + 1,6° (c=2, CHCl₃).

## Patentansprüche

1. Enantioselektives enzymatisches Verfahren zur Herstellung aliphatischer (S)-Cyanhydrine, in Gegenwart der (S)-Oxynitrilase aus Hevea brasiliensis, dadurch gekennzeichnet, daß ein aliphatischer Aldehyd oder ein unsymmetrisches aliphatisches Keton mit Blausäure in einem Verdünnungsmittel bei Temperaturen bis höchstens 10 °C umgesetzt wird.

2. Verfahren nach Anspruch 1, dadurch gekennzeichnet, daß ein aliphatischer, geradkettiger gesättigter oder ungesättigter Aldehyd mit 2 bis 12 C-Atomen eingesetzt wird.

3. Verfahren nach Anspruch 1, dadurch gekennzeichnet, daß ein aliphatisches, geradkettiges gesättigtes oder ungesättigtes Methylketon mit 2 bis 12 C-Atomen eingesetzt wird.

4. Verfahren nach Anspruch 1, dadurch gekennzeichnet, daß pro Mol Aldehyd oder Keton 2 bis 3 Mol Blausäure eingesetzt werden.

5. Verfahren nach Anspruch 1, dadurch gekennzeichnet, daß als Verdünnungsmittel eine wäßrige Pufferlösung eingesetzt wird.

6. Verfahren nach Anspruch 1, dadurch gekennzeichnet, daß die Reaktion bei Temperaturen von -5 bis 5 °C durchgeführt wird.

7. Verfahren nach Anspruch 1, dadurch gekennzeichnet, daß die Umsetzung in Gegenwart von 10⁴ bis 10⁶ IU der (S)-Oxynitrilase aus Hevea brasiliensis pro Mol Aldehyd oder Keton durchgeführt wird.

8. Verwendung der (S)-Oxynitrilase aus Hevea brasiliensis zur Herstellung von aliphatischen (S)-Cyanhydrinen aus aliphatischen Aldehyden oder unsymme-trischen aliphatischen Ketonen in Gegenwart von Blausäure.

## Claims

1. Enantioselective enzymic process for preparing aliphatic (S)-cyanohydrins in the presence of the (S)-oxynitrilase from Hevea brasiliensis, characterized in that an aliphatic aldehyde or an asymmetrical aliphatic ketone is reacted with hydrocyanic acid in a diluent at temperatures of up to at most 10°C.

2. Process according to Claim 1, characterized in that an aliphatic, straight-chain, saturated or unsaturated aldehyde having from 2 to 12 C atoms is employed.

3. Process according to Claim 1, characterized in that an aliphatic, straight-chain, saturated or unsaturated methyl ketone having from 2 to 12 C atoms is employed.

4. Process according to Claim 1, characterized in that from 2 to 3 mol of hydrocyanic acid are employed per mol of aldehyde or ketone.

5. Process according to Claim 1, characterized in that an aqueous solution of buffer is employed as the diluent.

6. Process according to Claim 1, characterized in that the reaction is carried out at temperatures of from -5 to 5°C.

7. Process according to Claim 1, characterized in that the reaction is carried out in the presence of from 10⁴ to 10⁶ IU of the (S)-oxynitrilase from Hevea brasiliensis per mol of aldehyde or ketone.

8. Use of the (S)-oxynitrilase from Hevea brasiliensis for preparing aliphatic (S)-cyanohydrins from aliphatic aldehydes or asymmetrical, aliphatic ketones in the presence of hydrocyanic acid.

## Revendications

1. Procédé enzymatique énantiosélectif pour la préparation de (S)-cyanhydrines aliphatiques en présence de (SI-oxynitrilase de *Hevea brasiliensis,* caractérisé en ce qu'on fait réagir un aldéhyde aliphatique ou une cétone aliphatique asymétrique avec de l'acide cyanhydrique dans un diluant à des températures allant jusqu'à 10°C au plus.

2. Procédé selon la revendication 1, caractérisé en ce qu'on utilise un aldéhyde aliphatique linéaire, saturé ou insaturé, ayant de 2 à 12 atomes de carbone.

3. Procédé selon la revendication 1, caractérisé en ce qu'on utilise une méthylcétone aliphatique linéaire, saturée ou insaturée, ayant de 2 à 12 atomes de carbone.

4. Procédé selon la revendication 1, caractérisé en ce qu'on utilise 2 à 3 moles d'acide cyanhydrique par mole d'aldéhyde ou de cétone.

5. Procédé selon la revendication 1, caractérisé en ce qu'on utilise une solution aqueuse tamponnée en tant que diluant.

6. Procédé selon la revendication 1, caractérisé en ce qu'on effectue la réaction à des températures de -5 à 5°C.

7. Procédé selon la revendication 1, caractérisé en ce qu'on effectue la réaction en présence de 10⁴ à 10⁶ UI de (S)-oxynitrilase de *Hevea Brasiliensis* par mole d'aldéhyde ou de cétone.

8. Utilisation de la (S)-oxynitrilase de *Hevea Brasiliensis* pour la préparation de (S)-cyanhydrines aliphatiques à partir d'aldéhydes aliphatiques ou de cétones aliphatiques asymétriques en présence d'acide cyanhydrique.
